# EUROPÄISCHE PATENTSCHRIFT

(11) **EP 0 398 935 B1**
(45) Veröffentlichungstag und Bekanntmachung des Hinweises auf die Patenterteilung: **10.08.1994**
(21) Anmeldenummer: 89901933.5
(22) Anmeldetag: 01.02.1989
(51) Int. Cl.: A61K 49/00

(54) **ULTRASCHALLKONTRASTMITTEL, VERFAHREN ZU DEREN HERSTELLUNG UND DEREN VERWENDUNG ALS DIAGNOSTIKA UND THERAPEUTIKA**
ULTRASONIC CONTRAST AGENTS, PROCESS FOR PRODUCING THEM AND THEIR USE AS DIAGNOSTIC AND THERAPEUTIC AGENTS
AGENTS DE CONTRASTE ULTRASONORES, PROCEDE POUR LEUR FABRICATION ET LEUR EMPLOI A TITRE D'AGENTS DIAGNOSTIQUES ET THERAPEUTIQUES

(30) Priorität: 05.02.1988 DE 3803972; 05.02.1988 DE 3803971
(43) Veröffentlichungstag der Anmeldung: 28.11.1990
(60) Teilanmeldung: 93112378.0
(73) Patentinhaber: SCHERING AKTIENGESELLSCHAFT, 13342 Berlin (DE)
(72) Erfinder: STEIN, Michael, D-1000 Berlin 31 (DE); HELDMANN, Dieter, D-1000 Berlin 51 (DE); FRITZSCH, Thomas, D-1000 Berlin 41 (DE); SIEGERT, Joachim, D-1000 Berlin 65 (DE); RÖSSLING, Georg, D-1000 Berlin 28 (DE); SPECK, Ulrich, D-1000 Berlin 28 (DE)
(74) Vertreter: Lederer, Franz, Dr.
(86) Internationale Anmeldenummer: DE8900069
(87) Internationale Veröffentlichungsnummer: WO8906978

(56) Entgegenhaltungen:
- EP-A- 123 235
- WO-A-80/02365
- DE-A- 3 341 001
- FR-A- 2 429 616
- FR-A- 2 496 460

## Beschreibung

Die Erfindung betrifft Mikropartikel nach dem Oberbegriff des Patentanspruchs 1, Verfahren zu deren Herstellung und deren Verwendung als Diagnostika und Therapeutika.

Es ist bekannt, daß durch periphere Injektion von Lösungen, die feine Gasblasen enthalten, cardiale Echokontraste erzielt werden können (Roelandt J, Ultrasound Med Biol 8:471-492, 1982). Diese Gasblasen werden in physiologisch verträglichen Lösungen z. B. durch Schütteln, andere Agitation oder durch Zusatz von Kohlendioxid erhalten. Sie sind jedoch hinsichtlich Anzahl und Größe nicht standardisiert und können nur unzulänglich reproduziert werden. Auch sind sie in der Regel nicht stabilisiert, so daß ihre Lebensdauer gering ist. Ihre mittleren Durchmesser liegen meist über Erythrocytengröße, so daß keine Lungenkapillarpassage mit nachfolgender Konstrastierung von Organen wie linkes Herz, Leber, Niere oder Milz möglich ist. Darüberhinaus eignen sie sich nicht für Quantifizierungen, da sich das von ihnen erzeugte Ultraschallecho aus mehreren, nicht voneinander zu trennenden Prozessen wie Blasenentstehung, Koaleszenz und Auflösung zusammensetzt. So ist es z. B. nicht möglich, mit Hilfe dieser Ultraschall-Kontrastmittel über die Messung des Kontrastverlaufs im Myokard Aussagen über die Transitzeiten zu gewinnen. Hierzu sind Kontrastmittel notwendig, deren Streukörper keiner eigenen Kinetik unterliegen.

Daneben gibt es Ultraschallkontrastmittel in Form von Partikeln (Ophir, Gobuty, Mc Whirt, Maklad, Ultrasonic Backscatter from Contrast-producing Collagen Microspheres, Ultrasonic Imaging 2:66-67, 1980). Ferner werden (Ophir, Mc Whirt, Maklad, Aqueous Solutions as Potential Ultrasonic Contrast Agents, Ultrasonic Imaging 1:265-279, 1979 sowie Tyler, Ophir, Maklad, In-vivo Enhancement of Ultrasonic Image Luminance by Aqueous Solutions with High Speed of Sound, Ultrasonic Imaging 3:323-329, 1981) Lösungen höherer Dichte als Ultraschall-Kontrastmittel eingesetzt. Es ist auch bekannt, Emulsionen als Ultraschall-Kontrastmittel zu verwenden (Mattrey, Andre, Ultrasonic Enhancement of Myocardial Infarction with Perfluorcarbon Compounds in Dogs, Am J Cardiol 54:206-210, 1984).

Es hat sich gezeigt, daß die gasfreien Kontrastmittel insgesamt nur eine geringe Effizienz besitzen. Die gashaltigen Zubereitungen haben den Nachteil einer nur geringen in-vivo Stabilität. Darüberhinaus ist die Größe der Gasblasen meistens nicht standardisierbar. Ausreichende Kontrasteffekte sind im arteriellen Gefäßsystem nach peripher venöser Injektion in aller Regel nicht möglich.

In den EP A2 123 235 und 0 122 624 werden Gasbläschen enthaltende Ultraschall-Kontrastmittel beschrieben, die die Lungenkapillaren passieren können und damit den gewünschten Kontrasteffekt bewirken.

Die EP A2 0 224 934 beschreibt Ultraschall-Kontrastmittel in Form von gasgefüllten Gelatine- oder Albuminhohlkörpern. Nachteilig ist jedoch die Verwendung von körperfremden oder denaturierten körpereigenen Eiweißen wegen des damit verbundenen allergenen Risikos.

Mit keinem der bisher bekannten Ultraschallkontrastmittel gelingt eine Organdarstellung mit ausreichender Signalintensität durch selektive Anreicherung nach i.v. Gabe. Quantifizierungen sind daher z.Z. nicht möglich.

Der Erfindung liegt die Aufgabe zugrunde, Ultraschallkontrastmittel auf der Basis von Mikropartikeln herzustellen, die neben bestimm- und reproduzierbaren Volumina eine erheblich längere Lebensdauer, als bisher bekannt, aufweisen, gute Verträglichkeit ohne allergenes Potential besitzen und intrazellulär in RES und damit auch in der Leber oder Milz angereichert werden können.

Erfindungsgemäß wird diese Aufgabe durch Mikropartikel, die aus einem synthetischen, bioabbaubaren Polymeren und einem Gas und/oder einer Flüssigkeit mit einem Siedepunkt unter 60° C bestehen, gelöst.

Als synthetische, bioabbaubare Polymere sind Polyester von α-, β-, γ- oder ε-Hydroxycarbonsäuren, Polyalkylcyanoacrylate, Polyaminosäuren, Polyamide, polyacrylierte Saccharide oder Polyorthoester zu nennen.

Als besonders geeignet haben sich
Polymilchsäure,
Poly-ε-caprolacton,
Copolymeres aus Milchsäure und Glykolsäure oder
ε-caprolacton,
Polyhydroxybuttersaüre,
Polyhydroxyvaleriansäure
Copolymeres aus Hydroxybutter- und Hydroxyvaleriansäure
Polymere aus Glutaminsäure und/oder Lysin,
Polydioxanon
Polymeres oder Copolymeres aus Aminosäuren oder/und
Terephthalsäure, Phthalsäure oder Sebacylsäure,
Polyacryldextran,
Polyacrylstärke,
Polyacrylamid,
Polyurethan,
Polyester,
Polyacetal,
Polyaminotriazol oder
Polyalkylcyanoacrylate
erwiesen.

Die Mikropartikel enthalten Gase und/oder Flüssigkeiten mit einem Siedepunkt unter 60° C in freier oder gebundener Form. Die Verwendung eines Gas-Flüssigkeits-Gemisches in den Ultraschall-Kontrastmitteln ist ebenfalls möglich.

Als Gase können beispielsweise Luft, Stickstoff, Edelgase, Wasserstoff, Kohlendioxid, Ammoniak, Sauerstoff, Methan, Ethan, Propan, Butan, Ethylen oder andere Kohlenwasserstoffe oder deren Gemische verwendet werden.

Als einschließbare Flüssigkeiten werden bevorzugt
1.1-Dichlorethylen,
2-Methyl-2-buten,
Isopropylchlorid,
2-Methyl-1.3-butadien,
2-Butin,
2-Methyl-1-buten,
Dibromdifluormethan,
Furan,
3-Methyl-1-buten,
Isopentan,
Diethylether,
3.3-Dimethyl-1-butin,
Dimethylaminoaceton,
Propylenoxid,
N-Ethylmethylamin,
Brommethan,
N-Ethyldimethylamin,
Methylenchlorid,
Pentan,
Cyclopentan,
2,3-Pentadien,
Cyclopenten
oder deren Gemische verwendet.

Mit Vorteil können die Mikropartikel auch Substanzen mit niedrigen Dampfdrücken und/oder niedrigen Siedepunkten, insbesondere ätherische Öle enthalten.

Durch Zugabe osmotisch aktiver Substanzen, beispielsweise Kochsalz, Galaktose, Glukose, Fruktose kann die physiologische Isotonie eingestellt werden.

Die Erfindung betrifft auch Verfahren zur Herstellung von Ultraschallkontrastmitteln, die aus synthetischen, bioabbaubaren Polymeren bestehen.

Ein vorteilhaftes Verfahren zur Herstellung der erfindungsgemäßen Ultraschallkontrastmittel besteht darin, daß ein Polymer oder ein Copolymer in einem oder mehreren mit Wasser nicht mischbaren, organischen Lösungsmitteln gelöst und anschließend ggf. nach Zusatz eines weiteren Lösungsmittels in Wasser emulgiert wird und die erhaltende Emulsion anschließend filtriert, ggf. getrocknet wird.

Ein weiteres Verfahren besteht darin, daß ein Polymer oder ein Copolymer in einem oder mehreren Gasblasen enthaltenden Lösungsmitteln gelöst und anschließend ggf. nach Zusatz eines weiteren Lösungsmittels oder eines weiteren Polymeren ausfällt oder in Wasser emulgiert werden und die erhaltende Suspension oder Emulsion anschließend filtriert, ggf. getrocknet wird. Zur Aufarbeitung ist auch das Gefriertrocknungsverfahren geeignet.

Mit Vorteil können die erhaltenen Produkte fein gemahlen werden.

Bei den beschriebenen Verfahren werden als Lösungsmittel beispielsweise Furan, Pentan, Aceton, Dioxan, Ethylacetat, Xylol, Methylenchlorid, Cyclohexan oder Hexan oder Lösungsmittelgemische verwendet. Der Emulsion können auch Emulgatoren zugesetzt werden.

In einer anderen Variante des Herstellungsverfahrens wird nicht von einem Polymeren ausgegangen, sondern von Monomeren, aus denen das Polymer gebildet wird. Dabei wird so gearbeitet, daß ein Monomer in einem oder mehreren organischen Lösungsmitteln gelöst und in 5 - 30 Teilen Wasser oder 0,01 - 0,1 N Salzsäure ggf. unter Zusatz von Emulgatoren oder Puffersubstanzen bei einer Temperatur unterhalb des Siedepunkts des organischen Lösungsmittels emulgiert wird und dieser Emulsion eine 0,2%- - 20%ige wäßrige Lösung eines zweiten Monomeren oder ggf. die Lösung einer pH-Wert erhöhenden Substanz zugegeben und ggf. getrocknet wird.

Bei einer anderen Arbeitsweise wird ein Monomer in einer oder mehreren gasblasenenthaltenden Flüssigkeiten ggf. unter Zusatz von Emulgatoren oder Puffersubstanzen gelöst oder dispergiert. Zu dieser Lösung oder Dispersion wird ggf. eine 0,2%- - 20%ige Lösung eines zweiten Monomeren oder eine pH-Wert erhöhende Substanz in gelöster oder gasförmiger Form gegeben und ggf. getrocknet.

Beispielsweise wird als erstes Monomer Terephthaloyl- oder Sebacoylchlorid oder Cyanacrylsäureester, als zweites Monomer L-Lysin und als organisches Lösungsmittel beispielsweise 2-Methyl-1.3-butadien, Dioxan, Methylenchlorid, Toluol oder Cyclohexan verwendet.

Gemäß einem weiteren Verfahren werden die Ultraschallkontrastmittel dadurch hergestellt, daß in einer 0,5- - 10%igen wäßrigen Lösung oder Dispersion eines Monomeren, die ggf. Zusätze wie Emulgatoren (0,01 - 5 %) oder Quasiemulgatoren (0,1 - 5 %) enthält, Gasblasen erzeugt und danach eine quervernetzende Substanz und/oder ein Reaktionsstarter zugesetzt werden.

Die im vorstehend beschriebenen Ultraschallkontrastmittel können sowohl für diagnostische als auch für therapeutische Verfahren verwendet werden.

Die Applikation der Mittel erfolgt beispielsweise durch Injektionen.

Die Erfindung wird durch folgende Beispiele erläutert:

### Beispiel 1:

500 mg Polylactid werden in 4 ml Furan und 0,6 ml Cyclohexan gelöst und diese Lösung in 40 ml einer 0,1%igen Lösung von Polyoxyethylenpolyoxypropylen-Polymer mit Molekulargewicht 12.000 (Pluronic® F 127) enhält, emulgiert, wobei die Temperatur während des Emulgierens unter 15° C gehalten wird. Die Temperatur wird anschließend zur Verdampfung des organischen Lösungsmittels langsam erhöht. Anschließend wird die entstandene Suspension gefriergetrocknet.

### Beispiel 2:

300 mg α-Cyanacrylsäurebutylester werden in 1 ml Furan gelöst und diese Lösung in 10 ml 0,1 N Salzsäure, die 1 % Polyoxyethylenpolyoxypropylen-Polymer mit Molekulargewicht 12.000 (Pluronic® F127) enthält, emulgiert, wobei die Temperatur während des Emulgierens unter 15° C gehalten wird. Nach Abschluß der Polymerisation wird die entstandene Suspension gefriergetrocknet.

### Beispiel 3:

200 mg α-Cyanacrylsäurebutylester werden in 0,4 ml Isopren gelöst und in 30 ml 0,01 N Salzsäure, die 1 % Polyoxyethylenpolyoxypropylen-Polymer mit Molekulargewicht 8.350 (Pluronic® F68) enthält, emulgiert, wobei die Temperatur während des Emulgierens unter 10° C gehalten wird. Nach Abschluß der Polymerisation wird die Suspension mit 0,1 N NaOH neutralisiert und mit Natriumchlorid isotonisiert.

### Beispiel 4:

400 mg α-Cyanacrylsäurebutylester werden in 0,4 ml Methylenchlorid gelöst und in 60 ml 0,01 N Salzsäure, die 1 % Polyoxyethylenpolyoxypropylen-Polymer mit Molekulargewicht 12.000 (Pluronic® F127) enthält, emulgiert, wobei die Temperatur während des Emulgierens unter 10° C gehalten wird. Nach Abschluß der Polymerisation wird die Suspension mit 0,1 N Natronlauge neutralisiert und mit Natriumchlorid isotonisiert.

### Beispiel 5:

400 mg Polycaprolacton werden in 6 ml Furan und 0,3 ml Cyclohexan gelöst und in 60 ml 1% Polyoxyethylenpolyoxypropylen-Polymer mit Molekulargewicht 12.000 (Pluronic® F127) emulgiert, wobei die Temperatur unter 15°C gehalten wird. Die Temperatur wird anschließend zur Verdampfung des organischen Lösungsmittels langsam erhöht. Danach wird die entstandene Suspension gefriergetrocknet.

### Beispiel 6:

400 mg Terephthalsäuredichlorid werden in 2 ml Furan gelöst und in 50 ml 3%iger Natriumcarbonatlösung, die 0,1 % Polyoxyethylenpolyoxypropylen-Polymer mit Molekulargewicht 12.000 (Pluronic® F127) enthält, emulgiert Nach Zusatz von 60 mg L-Lysin, in 5 ml 0,1 %iger Pluronic F127 gelöst, werden die Mikrokapseln zentrifugiert und mehrmals mit 0,1 %iger Pluronic F127 Lösung gewaschen. Vor Gebrauch wird die Suspension mit Natriumchlorid isotonisiert.

## Patentansprüche

1. Ultraschallkontrastmittel bestehend aus Mikropartikeln
dadurch gekennzeichnet, daß
die Mikropartikel aus synthetischen bioabbaubaren Polymeren und einem Gas und/oder einer Flüssigkeit mit einem Siedepunkt unter 60° C bestehen.

2. Ultraschallkontrastmittel nach Anspruch 1,
dadurch gekennzeichnet, daß
die Mikropartikel als synthetische bioabbaubare Polymere Polyester von α, β, γ oder ε-Hydroxycarbonsäuren, Polyalkylcyanoacrylate, Polyaminosäuren, Polyamide, Polyacrylierte Saccharide oder Polyorthoester enthalten.

3. Ultraschallkontrastmittel nach mindestens einem der Ansprüche 1 - 2,
dadurch gekennzeichnet, daß
die Mikropartikel als organische Flüssigkeiten mit einem Siedepunkt unter 60°C
1.1-Dichlorethylen,
2-Methyl-2-buten,
Isopropylchlorid,
2-Methyl-1.3-butadien,
2-Butin,
2-Methyl-1-buten,
Dibromdifluormethan,
Furan,
3-Methyl-1-buten,
Isopentan,
Diethylether,
3.3-Dimethyl-1-butin,
Dimethylaminoaceton,
Propylenoxid,
N-Ethylmethylamin,
Brommethan,
N-Ethyldimethylamin,
Methylenchlorid,
Pentan,
Cyclopentan,
2.3.-Pentadien,
Cyclopenten
oder deren Gemische enthalten.

4. Ultraschallkontrastmittel nach mindestens einem der Ansprüche 1 - 2,
dadurch gekennzeichnet, daß
die Mikropartikel als Gase
Luft,
Edelgase,
Stickstoff,
Sauerstoff,
Kohlendioxid,
Wasserstoff,
Ammoniak,
Ethylen,
Methan,
Ethan,
Propan oder
Butan
oder deren Gemische enthalten.

5. Ultraschallkontrastmittel nach mindestens einem der Ansprüche 1 - 4,
dadurch gekennzeichnet, daß
durch Zugabe osmotisch aktiver Substanzen, insbesondere Kochsalz, Mannit, Galaktose, Glukose, Fruktose, die physiologische Isotonie eingestellt ist.

6. Verfahren zur Herstellung von Ultraschallkontrastmitteln mit Mikropartikeln aus synthetischen bioabbaubaren Polymeren nach einem der Ansprüche 1 oder 2 - 4,
dadurch gekennzeichnet, daß
ein Polymer oder Copolymer in einem oder mehreren, mit Wasser nicht mischbaren, organischen Lösungsmitteln gelöst und anschließend ggf. nach Zusatz eines weiteren Lösungsmittels in Wasser emulgiert wird und die erhaltene Emulsion anschließend filtriert, ggf. getrocknet wird.

7. Verfahren zur Herstellung von Ultraschallkontrastmitteln mit Mikropartikeln aus synthetischen bioabbaubaren Polymeren nach einem der Ansprüche 1 - 4,
dadurch gekennzeichnet, daß
ein Polymer oder Copolymer in einem oder mehreren, Gasblasen enthaltenden Lösungsmittel gelöst und anschließend ggf. nach Zusatz eines weiteren Lösungsmittels oder eines weiteren Polymeren ausgefällt oder in Wasser emulgiert und die erhaltene Suspension oder Emulsion anschließend filtriert, ggf. getrocknet wird.

8. Verfahren nach Anspruch 6 oder 7
dadurch gekennzeichnet, daß
als Lösungsmittel Furan, Pentan, Aceton, Dioxan, Ethylacetat, p-Xylol, Methylenchlorid, Cyclohexan oder n-Hexan oder ein daraus bestehendes Lösungsmittelgemisch verwendet wird.

9. Verfahren nach Anspruch 6 oder 7
dadurch gekennzeichnet, daß
der Emulsion ein Emulgator zugesetzt wird.

10. Verfahren zur Herstellung von Ultraschallkontrastmitteln mit Mikropartikeln aus synthetischen, bioabbaubaren Polymeren nach einem der Ansprüche 1 und/oder 2 - 4,
dadurch gekennzeichnet, daß
ein Monomer in einem oder mehreren organischen Lösungsmitteln gelöst und in 5 - 30 Teilen Wasser oder 0,01 - 0,1 N Salzsäure ggf. unter Zusatz von Emulgatoren oder Puffersubstanzen bei einer Temperatur unterhalb des Siedepunkts des organischen Lösungsmittels emulgiert wird und dieser Emulsion eine 0,2% - 20%ige wäßrige Lösung eines zweiten Monomeren oder ggf. die Lösung einer pH-Wert erhöhenden Substanz zugegeben und ggf. getrocknet wird.

11. Verfahren zur Herstellung von Ultraschallkontrastmitteln mit Mikropartikeln aus synthetischen, bioabbaubaren Polymeren nach einem der Ansprüche 1 und/oder 2 - 4
dadurch gekennzeichnet, daß
ein Monomer in einer oder mehreren, gasblasenenthaltenden Flüssigkeiten ggf. unter Zusatz von Emulgatoren und/oder Puffersubstanzen gelöst oder dispergiert wird und dieser Lösung oder Dispersion ggf. eine 0,2% - 20%ige Lösung eines zweiten Monomeren oder eine pH-Wert erhöhende Substanz in gelöster oder gasförmiger Form zugegeben und ggf. getrocknet werden.

12. Verfahren nach Anspruch 10 oder 11
dadurch gekennzeichnet, daß
als erstes Monomer Terephthaloyl- oder Sebacoylchlorid oder Cyanacrylsäureester, als zweites Monomer L-Lysin und als organisches Lösungsmittel 2-Methyl-1.3-butadien, Methylenchlorid, Toluol, Dioxan oder Cyclohexan verwendet wird.

13. Verfahren zur Herstellung von Ultraschallkontrastmittel mit Mikropartikeln aus synthetischen bioabbaubaren Polymeren nach einem der Ansprüche 1 und/oder 2 - 4
dadurch gekennzeichnet, daß
in einer 0,5- - 10%igen wäßrigen Lösung eines Monomeren, die ggf. Zusätze wie Emulgatoren (0,01 - 5 %) oder Quasiemulgatoren (0,1 - 5 %) enthält, Gasblasen erzeugt und danach eine quervernetzende Substanz und/oder ein Reaktionsstarter zugesetzt werden.

14. Verwendung von Mikropartikeln gemäß den Ansprüchen 1 - 5 enthaltend ein Gas oder eine organische Flüssigkeit und synthetische bioabbaubare Polymere Zur Herstellung eines Präparates für die Ultraschalldiagnostik oder -therapie.

## Claims

1. Ultrasound contrast media consisting of microparticles, characterised in that the microparticles consist of synthetic bio-degradable polymers and a gas and/or a liquid having a boiling point below 60°C.

2. Ultrasound contrast media according to claim 1, characterised in that the microparticles contain as synthetic bio-degradable polymers polyesters of α-, β-, γ- or ε-hydroxycarboxylic acids, polyalkylcyanoacrylates, polyamino acids, polyamides, polyacrylated saccharides or polyorthoesters.

3. Ultrasound contrast media according to at least one of claims 1 and 2, characterised in that the microparticles contain as organic liquids having a boiling point below 60°C
1,1-dichloroethylene,
2-methyl-2-butene,
isopropyl chloride,
2-methyl-1,3-butadiene,
2-butyne,
2-methyl-1-butene,
dibromodifluoromethane,
furan,
3-methyl-1-butene,
isopentane,
diethyl ether,
3,3-dimethyl-1-butyne,
dimethylaminoacetone,
propylene oxide,
N-ethylmethylamine,
bromomethane,
N-ethyldimethylamine,
methylene chloride,
pentane,
cyclopentane,
2,3-pentadiene,
cyclopentene
or mixtures thereof.

4. Ultrasound contrast media according to at least one of claims 1 and 2, characterised in that the microparticles contain as gases
air,
noble gases,
nitrogen,
oxygen,
carbon dioxide,
hydrogen,
ammonia,
ethylene,
methane,
ethane,
propane or
butane
or mixtures thereof.

5. Ultrasound contrast media according to at least one of claims 1 to 4, characterised in that physiological isotonicity is obtained by the addition of osmotically active substances, especially sodium chloride, mannitol, galactose, glucose, fructose.

6. Process for the manufacture of ultrasound contrast media with microparticles of synthetic bio-degradable polymers according to any one of claims 1 or 2 to 4, characterised in that a polymer or copolymer is dissolved in one or more water-immiscible, organic solvents and subsequently, optionally after the addition of a further solvent, is emulsified in water, and the resulting emulsion is then filtered and, optionally, dried.

7. Process for the manufacture of ultrasound contrast media with microparticles of synthetic bio-degradable polymers according to any one of claims 1 to 4, characterised in that a polymer or copolymer is dissolved in one or more solvents containing gas bubbles and subsequently, optionally after the addition of a further solvent or a further polymer, is precipitated or is emulsified in water, and the resulting suspension or emulsion is then filtered and, optionally, dried.

8. Process according to claim 6 or 7, characterised in that there is used as solvent furan, pentane, acetone, dioxan, ethyl acetate, p-xylene, methylene chloride, cyclohexane or n-hexane or a solvent mixture consisting thereof.

9. Process according to claim 6 or 7, characterised in that an emulsifier is added to the emulsion.

10. Process for the manufacture of ultrasound contrast media with microparticles of synthetic bio-degradable polymers according to any one of claims 1 and/or 2 to 4, characterised in that a monomer is dissolved in one or more organic solvents and is emulsified in from 5 to 30 parts of water or 0.01 - 0.1N hydrochloric acid, optionally with the addition of emulsifiers or buffer substances, at a temperature below the boiling point of the organic solvent, and there is added to that emulsion a 0.2 % - 20 % aqueous solution of a second monomer or, optionally, a solution of a substance that increases the pH value, and, optionally, the emulsion is dried.

11. Process for the manufacture of ultrasound contrast media with microparticles of synthetic bio-degradable polymers according to any one of claims 1 and/or 2 to 4, characterised in that a monomer is dissolved or dispersed in one or more liquids containing gas bubbles, optionally with the addition of emulsifiers and/or buffer substances, and there is added to that solution or dispersion, optionally, a 0.2 % - 20 % aqueous solution of a second monomer or a substance that increases the pH value in dissolved or gaseous form, and, optionally, the solution or dispersion is dried.

12. Process according to claim 10 or 11, characterised in that there is used as the first monomer terephthaloyl or sebacoyl chloride or cyanoacrylic acid ester, as the second monomer L-lysine, and as the organic solvent 2-methyl-1,3-butadiene, methylene chloride, toluene, dioxan or cyclohexane.

13. Process for the manufacture of ultrasound contrast media with microparticles of synthetic bio-degradable polymers according to any one of claims 1 and/or 2 to 4, characterised in that gas bubbles are produced in a 0.5 % to 10 % aqueous solution of a monomer, which optionally contains additives such as emulsifiers (0.01 - 5 %) or quasi-emulsifiers (0.1 - 5 %), and thereafter a cross-linking substance and/or a reaction initiator are added.

14. The use of microparticles according to claims 1 to 5 conaining a gas or an organic liquid and synthetic bio-degradable polymers for the manufacture of a preparation for ultrasound diagnostics or therapy.

## Revendications

1. Agent de contraste pour ultrasons (échographies) constitués de microparticules, caractérisé en ce que les microparticules sont formées à partir de matières polymères synthétiques biodégradables et d'un gaz et/ou d'un liquide de point d'ébullition inférieur à 60 °C.

2. Agent de contraste selon la revendication 1, caractérisé en ce que les microparticules comprennent comme polymères synthétiques biodégradables des polyesters d'acides α, β, γ ou ε-hydroxycarboxyliques, des polycyanoacrylates d'alkyles, polyaminoacides, polyamides, saccharides (oses) polyacrylés ou polyorthoesters.

3. Agent de contraste selon la revendication 1 et/ou 2, caractérisé en ce que les microparticules comprennent, comme liquides organiques de point d'ébullition inférieur à 60 °C, l'un des suivants ou un mélange de plusieurs de ceux-ci :
1,1-Dichloréthylène,
2-Méthyl-2-butène,
Chlorure d'isopropyle,
2-Méthyl-1,3-butadiène,
2-Butyne,
2-Méthyl-1-butène,
Dibromodifluorométhane,
Furanne,
3-Méthyl-1-butène,
Isopentane,
Diéthyléther,
3,3-Diméthyl-1-butyne,
Diméthylaminoacétone,
Oxyde de propylène,
N-Ethylméthylamine,
Bromométhane,
N-Ethyldiméthylamine,
Chlorure de méthylène,
Pentane,
Cyclopentane,
2,3-Pentadiène,
Cyclopentène.

4. Agent de contraste selon la revendication 1 et/ou 2, caractérisé en ce que les microparticules comprennent comme gaz l'un des suivants ou un mélange de plusieurs de ceux-ci :
Air,
Gaz rares,
Azote,
Oxygène,
Dioxyde de carbone,
Hydrogène,
Ammoniac,
Ethylène,
Méthane,
Ethane,
Propane ou
Butane.

5. Agent de contraste selon une ou plusieurs des revendications 1 à 4, caractérisé en ce que son isotonie physiologique est établie avec des substances osmotiques, en particulier chlorure de sodium, mannitol, galactose, glucose ou fructose.

6. Procédé de préparation d'agents de contraste de microparticules selon l'une des revendications 1 et 2 à 4, procédé caractérisé en ce que l'on dissout un polymère ou un copolymère dans un ou plusieurs solvants organiques non-miscibles à l'eau, éventuellement après avoir ajouté un autre solvant on émulsionne la solution dans de l'eau puis on filtre l'émulsion formée et le cas échéant an la sèche.

7. Procédé de préparation d'agents de contraste de microparticules selon l'une des revendications 1 à 4, procédé caractérisé en ce que l'on dissout un polymère ou un copolymère dans un ou plusieurs solvants contenant des bulles gazeuses, éventuellement après avoir ajouté un autre solvant ou un autre polymère on le fait précipiter ou bien on émulsionne la solution dans de l'eau, puis on filtre la suspension ou l'émulsion formée et le cas échéant on la sèche.

8. Procédé selon la revendication 6 ou 7, caractérisé en ce que le solvant est pris parmi le furanne, le pentane, l'acétone, le dioxanne, l'acétate d'éthyle, le p-xylène, le chlorure de méthylène, le cyclohexane et le n-hexane ou parmi plusieurs de ces solvants à la fois.

9. Procédé selon la revendication 6 ou 7, caractérisé en ce que l'on ajoute un émulsionnant à l'émulsion.

10. Procédé de préparation d'agents de contraste de microparticules selon l'une des revendications 1 et 2 à 4, caractérisé en ce que l'on dissaut un monomère dans un ou plusieurs solvants organiques, on émulsionne la solution dans 5 à 30 parties d'eau ou d'acide chlorhydrique 0,01-0,1 N, éventuellement en présence d'émulsionnants ou de matières de tamponnage, à une température inférieure au point d'ébullition du solvant, puis on ajoute à l'émulsion une solution aqueuse à 0,2 % - 20 % d'un second monomère ou éventuellement une solution d'une substance élevant le pH, et le cas échéant on la sèche.

11. Procédé de préparation d'agents de contraste de microparticules selon l'une des revendications 1 et 2 à 4, procédé caractérisé en ce que l'on dissout ou disperse un monomère dans un ou plusieurs liquides contenant des bulles gazeuses, éventuellement en présence d'émulsionnants et/ou de matières de tamponnage, on ajoute éventuellement à la solution ou à la dispersion une solution à 0,2 % - 20 % d'un second monomère, ou bien une substance élevant le pH, en solution ou à l'état gazeux, et le cas échéant on la sèche.

12. Procédé selon la revendication 10 ou 11, caractérisé en ce que le premier monomère est le chlorure de téréphtaloyle ou de sébaçoyle ou un ester de l'acide cyanoacrylique, le second monomère est la L-lysine et le solvant organique le 2-méthyl-1,3-butadiène, le chlorure de méthylène, le toluène, le dioxanne ou le cyclohexane.

13. Procédé de préparation d'agents de contraste de microparticules selon l'une des revendications 1 et 2 à 4, procédé caractérisé en ce que l'on forme des bulles gazeuses dans une solution aqueuse à 0,5 - 10 % d'un monomère contenant éventuellement des additifs tels qu'émulsionnants (0,01 - 5 %) ou quasiémulsionnants (0,1 - 5 %), puis on ajoute une substance réticulante et/ou un déclencheur de réaction.

14. L'emploi de microparticules selon les revendications 1 à 5, contenant un gaz ou un liquide organique avec des matières polymères synthétiques biodégradables, pour en former une préparation en vue de diagnostics ou de traitements thérapeutiques par ultrasons.
